# EUROPEAN PATENT APPLICATION

(11) **EP 3 385 712 A1**
(43) Date of publication of application: **10.10.2018**
(21) Application number: 17164618.5
(22) Date of filing: 03.04.2017
(51) Int. Cl.: G01N 33/543, G01N 33/68, G01N 33/84

(54) **IMPROVED CALPROTECTIN ASSAY**

(71) Applicant: Gentian AS, 1509 Moss (NO)
(72) Inventor: Sundrehagen, Erling, SE-457 72 Grebbestad (SE); Sunde, Kathrin, NO-1534 Moss (NO); Nilsen, Tom, NO-1509 Moss (NO); David, Arnaud, NO-0585 Oslo (NO)
(74) Representative: Kransell & Wennborg KB

(57) **Abstract**

The concentration of calprotectin in a sample can be determined with improved accuracy using a particle enhanced turbidimetric immunoassay, said assay comprising anti-calprotectin antibodies or fragments thereof, immobilized to particles, a reaction buffer, and a calibrator comprising calprotectin in a buffer; wherein the choice of calibrator buffer and/or reaction buffer is determined by the character of the sample.

## Description

### Technical field

This disclosure relates to the field of diagnostic assays, and in particular to an improved assay method and kit for the determination of the concentration of calprotectin in a sample.

### Background

Calprotectin is a complex of the mammalian proteins S100A8 and S100A9, belonging to the S100 family. Calprotectin is expressed in neutrophils and monocytes. In the presence of calcium, the calprotectin complex is capable of sequestering the essential nutrients manganese and zinc via chelation. As a result, calprotectin exhibits antimicrobial properties and is believed to be an important part of the innate host defence mechanisms.

Calprotectin is said to exist in both dimeric, trimeric and tetrameric forms. As a dimer, calprotectin comprises the polypeptide chains S100A8 and S100A9. Human S100A8 consists of 93 amino acid residues and has a molecule weight of 10.8 kDa. S100A9 contains 113 amino acid residues and has a molecular weight of 13.2 kDa. Thus, in the dimeric form, calprotectin has a molecular weight of 24 kDa. As a trimer, calprotectin is a 36 kDa heterotrimeric protein with two heavy chains (13.2 kDa) and one light chain (10.8 kDa) non-covalently linked. As a tetramer, calprotectin is a 48 kDa heterotrimeric protein with two heavy chains (13.2 kDa) and two light chains (10.8 kDa) non-covalently linked. In addition, a truncated isoform of S100A9 exists, due to an alternative translation leading to the loss of the 4 first residues in N-terminal (J Am Soc Mass Spectrom. 1999 Nov;10(11):1124-30.) This has a molecular weight of 12.7 kDa. The incorporation of the truncated from into the dimeric form leads to the formation of a slightly smaller complex, with a molecular weight of 23.5 kDa. Examples of possible isoforms of calprotectin are listed in Table 1:

**Table 1. Molecular weight intervals for calprotectin isoforms**

| **Isoform** | **Molecular weight** |
|---|---|
| Monomer | From 10.8 to 13.2 kDa |
| Homodimer | From 21.6 to 26.4 kDa |
| Heterodimer | From 23.5 to 25.9 kDa |
| Trimer | From 32.4 to 39.6 kDa |
| Tetramer | From 43.2 to 52.8kDa |

Calprotectin has been used as a marker for neutrophil activation and has been shown to be elevated in inflammatory conditions where neutrophil activation is involved. Elevated levels of calprotectin have been detected in blood and faeces, and the presence of calprotectin is considered to be an important marker of inflammation.

A comprehensive review was published in 2016; M. Pruenster et al. S100A8/A9: From basic science to clinical application. Pharmacology. 2016-08-01, vol.167, p.120-131. It appears that plasmatic S100A8/A9 levels can be used as biomarkers of inflammation in a high number of inflammatory disorders. Faecal calprotectin is already widely used for differentiation of irritable bowel syndrome from inflammatory bowel disease and for monitoring disease activity in Crohn's disease and ulcerative colitis.

A study published in 2016 indicates a role of calprotectin as a biomarker in early sepsis (A. Larsson et al., Performance of plasma calprotectin as a biomarker of early sepsis: a pilot study, Biomarkers in Medicine, 14 July 2016, DOI: 10.2217/bmm-2016-0032).

There are indications that calprotectin is a local, site specific marker, and thus it becomes interesting to determine the level of calprotectin in different samples, such as urine, saliva, cerebrospinal fluid, synovial fluid and possibly also in biopsies.

Calprotectin can be determined in an enzyme-linked immunosorbent assay which is a plate-based assay technique using a capture antibody immobilized to a solid surface, and forming a complex with an antigen in the sample. This antigen will be detected by a specific antibody forming a complex. This complex will then be bound by a secondary antibody linked to an enzyme. Detection is accomplished by assessing the conjugated enzyme activity via incubation with a substrate to produce a measureable product. This is frequently referred to as a sandwich-ELISA. An ELISA is typically performed in 96-well (or 384-well) polystyrene plates, which will passively bind antibodies and proteins. Several ELISA-based tests for calprotectin are currently available on the market.

Calprotectin can also be determined turbidimetrically, which allows the automation of the assay to a degree not possible with ELISA-based assays. One example of a turbidimetric calprotectin assay has been disclosed in the patent application EP 1739430 A (AXIS SHIELD ASA).

Later, a new turbidimetric immunoasssay for fully automatized clinical analysers was disclosed by Nilsen, Tom, et al., A new turbidimetric immunoassay for serum calprotectin for fully automatized clinical analysers, Journal of Inflammation. 2015.07.25, vol.12, p.45-52.

With the increasing popularity of calprotectin assays, and their application to the diagnosis of a constantly growing number of diseases, there is need for ensuring the accuracy of the assay, regardless of type of sample and pretreatment of the sample.

### Summary

One objective is to make available an improved assay for the determination of calprotectin concentrations in biological samples, applicable to samples of different origin, and exhibiting increased accuracy and repeatability.

The present inventors have surprisingly found that not only does calprotectin exist in many different isoforms in its natural state, in the mammal body, it also significantly changes appearance when a sample is taken, depending on the handling and treatment of the sample.

Without wishing to be bound to any theory, the inventors postulate that calprotectin, being a central component in a non-specific innate host defense mechanism, has an elevated capacity of adapting to the environment. Nevertheless, there appears to be a general consensus that calprotectin is very stable. To the best knowledge of the present inventors, calprotectin standards used for calibrating existing assays, regardless of whether the assay is based on ELISA or turbidimetry, use the tetrameric form. This discrepancy may result in deviations between the true concentration and the detected concentration, which may introduce an uncertainty when the concentration of calprotectin is used for diagnosis, prediction or surveillance of different clinical disorders.

Thus, a first aspect of the invention relates to a method for determination of calprotectin in a sample using a particle enhanced turbidimetric immunoassay (PETIA), said assay comprising anti-calprotectin antibodies or fragments thereof, immobilized to particles, a reaction buffer, and a calibrator comprising calprotectin in a buffer; wherein the choice of calibrator buffer and/or reaction buffer is/are determined by the character of the sample.

According to an embodiment of said first aspect, the sample is a lithium heparin treated plasma sample or a serum sample and the calibrator buffer and/or reaction buffer comprises an excess of bivalent cations.

According to another embodiment of said first aspect, the sample is an EDTA treated sample and the calibrator buffer and/or reaction buffer comprises EDTA.

According to yet another embodiment of said first aspect, the sample is a fecal extract and the calibrator buffer and/or reaction buffer comprises EDTA.

A second aspect relates to a method for determination of calprotectin in a sample using a particle enhanced turbidimetric immunoassay (PETIA), said assay comprising anti-calprotectin antibodies or fragments thereof, immobilized to particles, a reaction buffer, and a calibrator comprising calprotectin in a buffer; wherein said calibrator buffer comprises EDTA and/or that EDTA is added to the sample before or simultaneously with the addition of the reaction buffer.

A third aspect relates to a method for determination of calprotectin in a sample using a particle enhanced turbidimetric immunoassay (PETIA), said assay comprising anti-calprotectin antibodies or fragments thereof, immobilized to particles, a reaction buffer, and a calibrator comprising calprotectin in a buffer; wherein said calibrator buffer comprises bivalent cations and/or that an excess of bivalent cations is added to the sample before or simultaneously with the addition of the reaction buffer.

According to embodiments of said first, second and third aspects, said anti-calprotectin antibodies or fragments thereof are antibodies against purified S100A8/A9 heterotetramers extracted from human granulocytes.

In the alternative, said anti-calprotectin antibodies or fragments thereof are antibodies against recombinant calprotectin.

According to an embodiment equally applicable to said first, second and third aspects, said particles are polystyrene particles having a uniform particle size in the interval of 60 - 180 nm, preferably 60 - 120 nm, most preferably 80 - 100 nm in diameter.

According to an embodiment equally applicable to said first, second and third aspects, and freely combinable with any of the above aspects and embodiments, said reaction buffer is chosen from phosphate buffered saline (PBS), 3-(N-morpholino) propanesulfonic (MOPS) buffer and (4-(2-hydroxyethyl)-1-piperazine ethanesulfonic acid (HEPES) buffer.

A fourth aspect relates to a kit, comprising one or more anti-calprotectin antibodies or fragments thereof immobilized on particles, a reaction buffer, and a calibrator comprising calprotectin in a buffer, wherein the reaction buffer is chosen from phosphate buffered saline (PBS), 3-(N-morpholino) propanesulfonic (MOPS) buffer and (4-(2-hydroxyethyl)-1-piperazine ethanesulfonic acid (HEPES) buffer, and in that the calibrator buffer comprises EDTA when the sample is an EDTA treated sample.

According to an embodiment of the fourth aspect, the calibrator buffer comprises EDTA when the sample is a fecal extract.

A fifth aspect relates to a kit comprising one or more anti-calprotectin antibodies or fragments thereof immobilized on particles, a reaction buffer, and a calibrator comprising calprotectin in a buffer, wherein the reaction buffer is chosen from phosphate buffered saline (PBS), 3-(N-morpholino) propanesulfonic (MOPS) buffer and (4-(2-hydroxyethyl)-1-piperazine ethanesulfonic acid (HEPES) buffer, and wherein the reaction buffer and the calibrator buffer comprise EDTA.

A sixth aspect relates to a kit comprising one or more anti-calprotectin antibodies or fragments thereof immobilized on particles, a reaction buffer, and a calibrator comprising calprotectin in a buffer, wherein the reaction buffer is chosen from phosphate buffered saline (PBS), 3-(N-morpholino) propanesulfonic (MOPS) buffer and (4-(2-hydroxyethyl)-1-piperazine ethanesulfonic acid (HEPES) buffer, and wherein the reaction buffer and the calibrator buffer comprise an excess of bivalent cations.

According to embodiments of said fourth, fifth and sixth aspects, said anti-calprotectin antibodies or fragments thereof are antibodies against purified S100A8/A9 heterotetramers extracted from human granulocytes.

In the alternative, or in combination therewith, said anti-calprotectin antibodies or fragments thereof are antibodies against recombinant calprotectin.

According to further embodiments of said fourth, fifth and sixth aspects, said particles are polystyrene particles having a uniform particle size in the interval of 60 - 180 nm, preferably 60 - 120 nm, most preferably 80 - 100 nm in diameter.

The foregoing summary is illustrative only and is not intended to be in any way limiting. In addition to the illustrative aspects, embodiments and features described above, further aspects, embodiments and features will become apparent by reference to the following drawings and the detailed description.

### Brief description of the drawings

The invention is now described, by way of example, with reference to the accompanying drawings, in which:
Figure 1 shows a boxplot demonstrating a difference between the sample materials based on measured calprotectin concentration in patient samples.
Figure 2 shows a bar plot of each individual patient and sample material,.
Figure 3 shows two pie charts illustrating the abundance of different proteins in blood.
Figure 4 is a histogram showing the molecular weight distribution of proteins in the plasma proteome.
Figure 5 shows the principle of the purification of patient samples, developed by the present inventors in order to isolate calprotectin from patient samples for SEC-MALLS/RI analysis. The method consists in a series of two steps of size exclusion chromatography, accompanied by parallel turbidimetric measurements to determine the presence of calprotectin. The resulting fraction, fraction 2, is then ready for SEC-MALLS/RI analysis. The fraction can also be divided in several sub-fractions if needed.
Figure 6 is a MALDI-Mass Spectrometry profile of native calprotectin, indicating the presence of three chains: S100A8 at 10838.3 Da, S100A9 at 13158.6 Da, and its truncated form at 12694.1 Da.

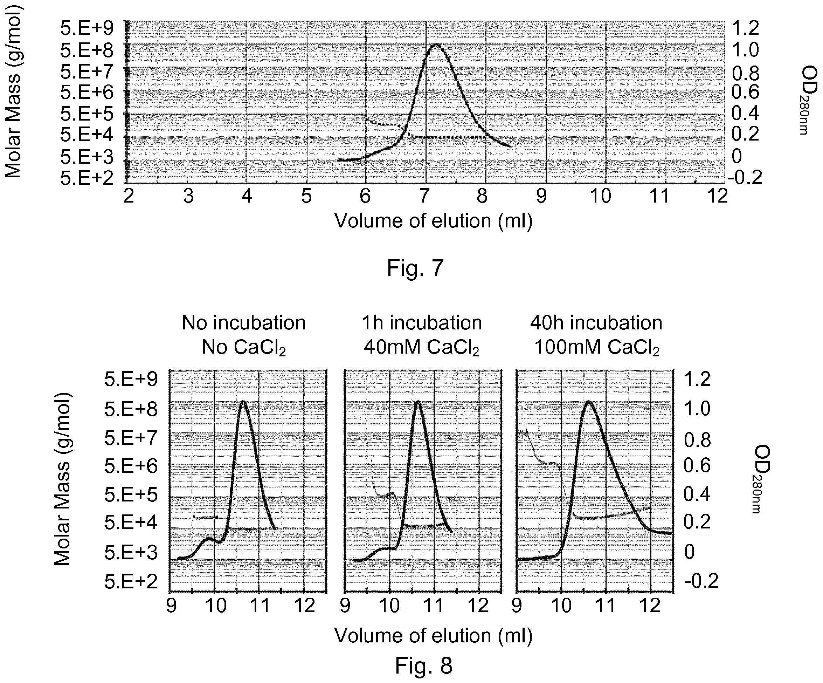

Figure 7 shows the SEC-MALLS/RI profile of native calprotectin, illustrating the presence of a main isoform at 45-49 kDa, indicating a S100A8 tetrameric form, and its aggregated forms at 120-160 kDa.
Figure 8 illustrates the effect of an addition of Ca²⁺ added as CaCl₂, to native calprotectin at time 0, after 1 h incubation and 40 h incubation. Analysis performed by SEC-MALLS/RI.

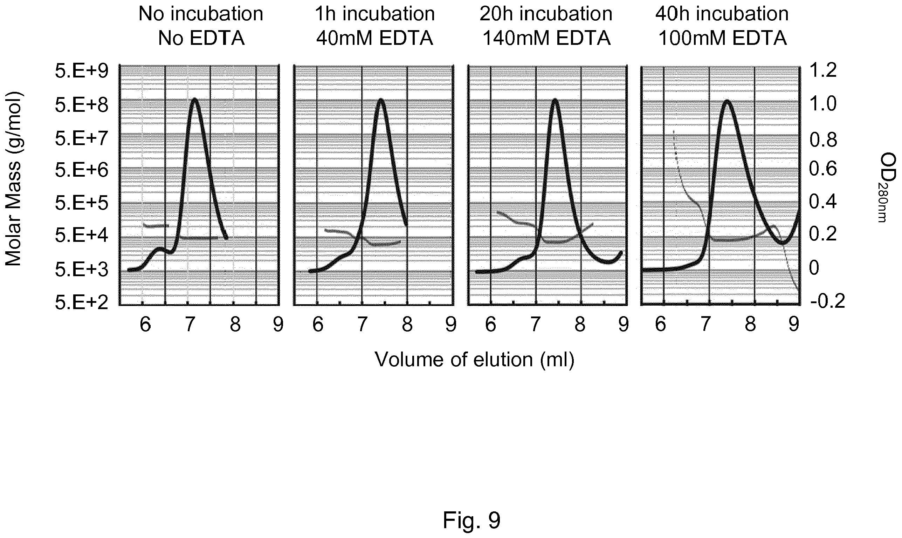

Figure 9 illustrates the effect of an addition of EDTA to native calprotectin at time 0, and after incubation for 1, 20 and 40 hours. Analysis performed by SEC-MALLS/RI.

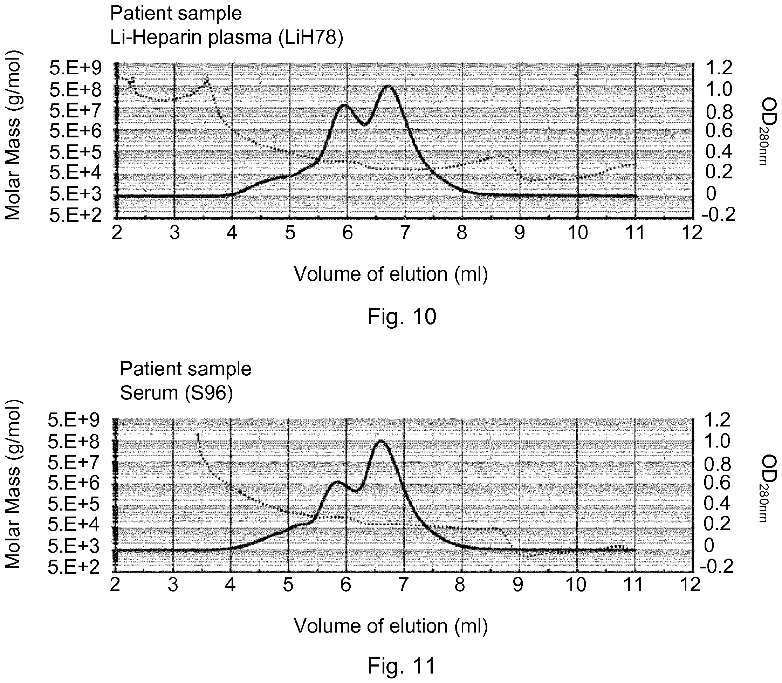

Figure 10 shows the SEC-MALLS/RI profile of fraction 2 of the lithium heparin plasma sample #78 after the double SEC-purification described in Fig. 5.
Figure 11 shows the SEC-MALLS/RI profile of fraction 2 of the serum sample #96 after the double SEC-purification described in Fig. 5.

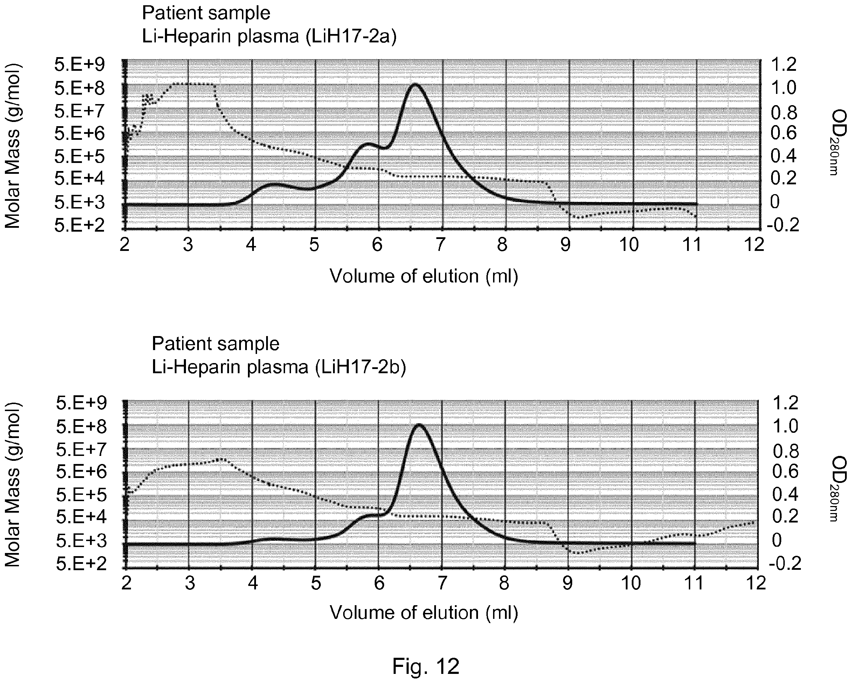

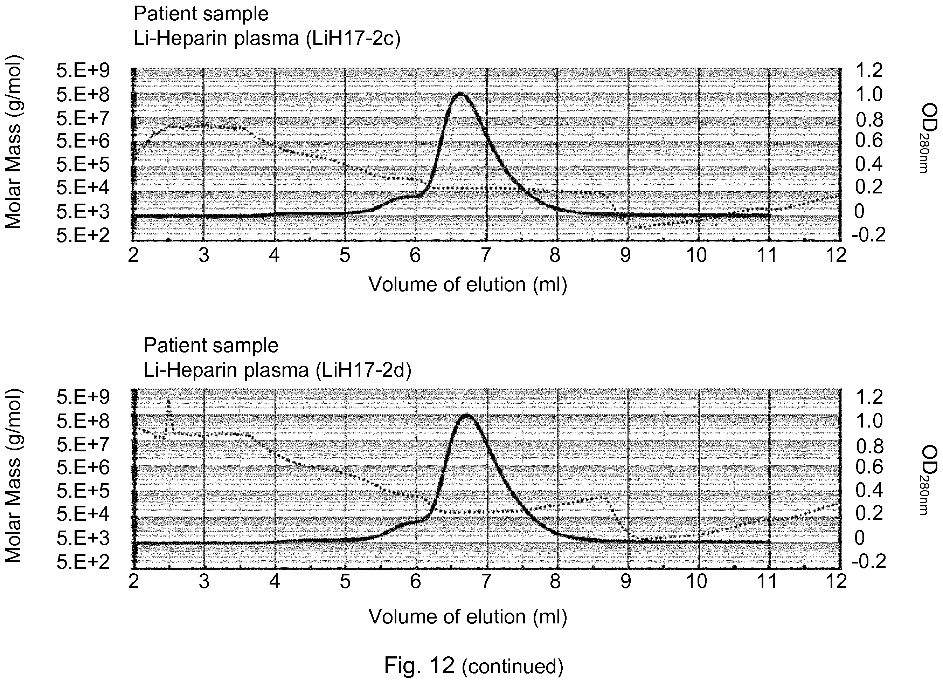

Figure 12 shows the SEC-MALLS/RI profiles of the 4 equal sub-fractions of fraction 2 of the Li-Heparin plasma sample #17 after the double SEC-purification described in Fig. 5.

### Detailed description

Before the present invention is described, it is to be understood that the terminology employed herein is used for the purpose of describing particular embodiments only and is not intended to be limiting, since the scope of the present invention will be limited only by the appended claims and equivalents thereof.

It must be noted that, as used in this specification and the appended claims, the singular forms "a," "an," and "the" include plural referents unless the context clearly dictates otherwise.

Calprotectin has been the subject of considerable research for several decades. A somewhat inconsistent vocabulary has developed as a result. For the avoidance of doubt, the term calprotectin is used herein synonymously with "calgranulin", "L1 protein, "MRP 8/14" and "cystic fibrosis associated antigen, CFA".

The term "immobilized" as in "anti-calprotectin antibodies or fragments thereof, immobilized to particles" includes both chemical bonds, such as covalent bonds, and physical adsorption.

Blood tests, involving laboratory examination of a sample of blood to obtain information about its physical and chemical properties, today form the foundation of medical diagnosis. Hundreds of haematological tests and procedures have been developed over the years. While the tests were originally performed manually, one by one, the field of clinical chemistry has become highly automated. Today multiple tests can be carried out simultaneously on one sample of blood using automated instruments known as autoanalyzers.

While the presently disclosed assay method can be performed manually, the assay is preferably performed on an autoanalyzer, as this allows high throughput, reduces error and minimizes staffing requirements. A closed, automated sample handling also minimizes the risk that staff is exposed to possible blood borne pathogens.

When taking a blood sample, blood is usually drawn from a vein in the arm, and collected in standardized sterile blood collection tubes. Different tubes are available, depending on which component or components that is to be analysed. A blood collection tube can be empty, or prefilled with a buffer or more frequently, with an anticoagulant. Examples of anticoagulants include sodium citrate, lithium-heparin, and EDTA. Different combinations are also possible, there are for example tubes containing sodium heparin and EDTA. The Vacutainer™ (BD, Becton, Dickinson and Company) is one example of blood collection tubes, available in different sizes and pre-filled with various reagents.

A distinction is made between plasma samples and serum samples. When a plasma sample is desired, blood is collected in a tube containing an anticoagulant, the tube is turned upside down about 5 to 10 times directly after filling, in order to ensure mixing. The tubes are then stored in an upright position in room temperature to avoid haemolysis. EDTA and lithium heparin containing tubes can generally be stored up to 4 hours in room temperature. Alternatively the tubes must be deep-frozen and stored for later analysis.

For obtaining a serum sample, blood is collected in an empty sample tube or a tube containing a coagulation activator. After filling, the tubes are turned upside down about 5 to 10 times directly after filling, in order to ensure mixing. The tubes are then stored in an upright position in room temperature to avoid haemolysis. In most cases, serum tubes can be stored in room temperature and without centrifuging for up to 4 hours. Upon arrival in the laboratory, the tubes are centrifuged, for example centrifuged for 10 minutes at 2000 x g. Following centrifugation, a layer of blood cells can be seen at the bottom of the tube, and the fluid above is called serum. The serum should be separated and stored cold without any additives until transport or analysis.

Specific routines and in some cases also specific collection tubes with corresponding buffers and additives have been developed for other samples. For the collection of urine, a collection tube containing a buffered boric acid solution is available. Sample collection tubes of the Vacutainer™ type can also be used for the collection of other fluids, such as cerebrospinal fluid, synovial fluid etc. For the collection of other samples, such as biopsies or faeces, standardized protocols already exist, involving steps of homogenization and extraction.

In all commercial assays, it is assumed that calprotectin is very stable. As a matter of fact, the stability of calprotectin is an important factor in the increasing popularity of calprotectin as a biomarker in a variety of inflammatory disorders. Little is however known about the appearance of calprotectin, i.e. the dominating isoform or isoforms in different samples, how these are influenced by the treatment of the sample, and if they change with time.

The present inventors surprisingly found that calprotectin is present in many different forms, and that the distribution of isoforms is dependent of time and sample treatment, as well as possibly other factors. The present inventors then determined that these differences have consequences for the measured concentration of calprotectin.

The results obtained so far show that there is a difference between the calprotectin concentrations determined in the different samples although they originate from the same patient. The most striking result is that the EDTA treated samples exhibited a significantly lower concentration than what was determined in parallel samples. There was also a difference between lithium heparin and serum sample, most apparent at lower concentrations. This is not acceptable.

Consequently a first aspect of the invention relates to a method for determination of calprotectin in a sample using a particle enhanced turbidimetric immunoassay (PETIA), said assay comprising anti-calprotectin antibodies or fragments thereof, immobilized to particles, a reaction buffer, and a calibrator comprising calprotectin in a buffer; wherein the choice of calibrator buffer is determined by the character of the sample.

According to an embodiment of said first aspect, the sample is a lithium heparin treated plasma sample or a serum sample and the calibrator buffer comprises an excess of bivalent cations.

According to another embodiment of said first aspect, the sample is an EDTA treated sample and the calibrator buffer comprises EDTA.

According to yet another embodiment of said first aspect, the sample is a fecal extract and the calibrator buffer comprises EDTA.

A second aspect relates to a method for determination of calprotectin in a sample using a particle enhanced turbidimetric immunoassay (PETIA), said assay comprising anti-calprotectin antibodies or fragments thereof, immobilized to particles, a reaction buffer, and a calibrator comprising calprotectin in a buffer; wherein said calibrator buffer comprises EDTA and that EDTA is added to the sample before or simultaneously with the addition of the reaction buffer.

A third aspect relates to a method for determination of calprotectin in a sample using a particle enhanced turbidimetric immunoassay (PETIA), said assay comprising anti-calprotectin antibodies or fragments thereof, immobilized to particles, a reaction buffer, and a calibrator comprising calprotectin in a buffer; wherein said calibrator buffer comprises bivalent cations and that an excess of bivalent cations is added to the sample before or simultaneously with the addition of the reaction buffer.

According to embodiments of said first, second and third aspects, said anti-calprotectin antibodies or fragments thereof are antibodies against purified S100A8/A9 heterotetramers (45 - 50 kDa) extracted from human granulocytes.

In the alternative, said anti-calprotectin antibodies or fragments thereof are antibodies against recombinant calprotectin available from commercial sources.

It is also conceived that a mixture of anti-calprotectin antibodies or fragments thereof are produced by immunizing a host using different isoforms of calprotectin.

According to an embodiment equally applicable to said first, second and third aspects, said particles are polystyrene particles having a uniform particle size in the interval of 60 - 180 nm, preferably 60 - 120 nm, most preferably 80 - 100 nm in diameter.

According to another embodiment, particles of two distinctly different sizes are used, wherein the total amount of particles comprises two populations. One population of particles preferably has a diameter which is 80 % or less of the diameter of a second population of particles. For example, where the second population of particles has a diameter of 100 nm or more, the first population of particles has a diameter of 80 nm or less. The two populations of particles are preferably equal in number, but is it also conceived that the smaller particles are present in a larger amount.

According to yet another embodiment, different antibodies are immobilized to the two particle populations. By the expression "different antibodies" is here meant antibodies raised against different forms of the same antigen, for example different isoforms of calprotectin.

According to an embodiment equally applicable to said first, second and third aspects, and freely combinable with any of the above aspects and embodiments, said reaction buffer is chosen from phosphate buffered saline (PBS), 3-(N-morpholino) propanesulfonic (MOPS) buffer and (4-(2-hydroxyethyl)-1-piperazine ethanesulfonic acid (HEPES) buffer.

A fourth aspect relates to a kit, comprising one or more anti-calprotectin antibodies or fragments thereof immobilized on particles, a reaction buffer, and a calibrator comprising calprotectin in a buffer, wherein the reaction buffer is chosen from phosphate buffered saline (PBS), 3-(N-morpholino) propanesulfonic (MOPS) buffer and (4-(2-hydroxyethyl)-1-piperazine ethanesulfonic acid (HEPES) buffer, and wherein the calibrator buffer comprises EDTA when the sample is an EDTA treated sample.

According to an embodiment of the fourth aspect, the calibrator buffer comprises EDTA when the sample is a fecal extract.

A fifth aspect relates to a kit comprising one or more anti-calprotectin antibodies or fragments thereof immobilized on particles, a reaction buffer, and a calibrator comprising calprotectin in a buffer, wherein the reaction buffer is chosen from phosphate buffered saline (PBS), 3-(N-morpholino) propanesulfonic (MOPS) buffer and (4-(2-hydroxyethyl)-1-piperazine ethanesulfonic acid (HEPES) buffer, and wherein the reaction buffer and/or the calibrator buffer comprise EDTA.

A sixth aspect relates to a kit comprising one or more anti-calprotectin antibodies or fragments thereof immobilized on particles, a reaction buffer, and a calibrator comprising calprotectin in a buffer, wherein the reaction buffer is chosen from phosphate buffered saline (PBS), 3-(N-morpholino) propanesulfonic (MOPS) buffer and (4-(2-hydroxyethyl)-1-piperazine ethanesulfonic acid (HEPES) buffer, and wherein the reaction buffer and/or the calibrator buffer comprise an excess of bivalent cations.

According to embodiments of said fourth, fifth and sixth aspects, said anti-calprotectin antibodies or fragments thereof are antibodies against purified S100A8/A9 heterotetramers extracted from human granulocytes.

In the alternative, or in combination therewith, said anti-calprotectin antibodies or fragments thereof are antibodies against recombinant calprotectin.

According to further embodiments of said fourth, fifth and sixth aspects, said particles are polystyrene particles having a uniform particle size in the interval of 80 - 200 nm, preferably 100 - 180 nm, most preferably 120 nm in diameter.

### Particle enhanced turbidimetric immunoassay

A particle enhanced turbidimetric immunoassay for the determination of calprotectin in serum has been developed by Gentian AS, Norway. The assay reagents consist of immunoparticles, buffer, calibrators and controls. The immunoparticles are prepared for example by covalently attaching purified avian immunoglobulin fractions to uniform polystyrene particles. Particles with a diameter in the interval 60 - 180 nm, preferably 60 - 120 nm, most preferably 80 - 100 nm in diameter.

### Antigens

The antigen can be either native antigen isolated from granulocytes in human blood, or recombinant antigen, produced for example in *E. coli.*

Methods for the isolation of granulocytes are known to persons skilled in the art. Anti-coagulant treated blood is obtained from healthy volunteers, and cells removed by centrifugation and resuspended in a suitable buffer. Granulocytes are isolated by Ficoll density gradient centrifugation. The granulocytes are then extracted and homogenized in a buffer, and approximately 90 % of the total calprotectin can be recovered in the supernatant following ultracentrifugation. Purification of the calprotectin can be performed using chromatography.

Recombinant human calprotectin can be obtained from different suppliers, for example ImmunDiagnostik AG, Germany; Abcam plc., UK; Yashraj Biotechnology Ltd, India or Hycult Biotech Inc., USA.

### Antibodies

Antibodies against calprotectin can be produced according to methods known to the skilled person, either in mammal hosts, e.g. goats or rabbits, or preferably in hens, in which case the antibodies can be isolated from the egg yolk.

Avian immunoglobulin fractions such as those used in this assay can be purified from eggs from immunized hens, immunized with purified S100A8/S100A9 tetramer extracted from human granulocytes. Recombinant calprotectin can also be used. The antigen and suitable adjuvant, e.g. complete Freund's adjuvant, is injected subcutaneously and/or intramuscularly into breast tissue of the hen. Immunizations are repeated at regular intervals, for example on day 10, 20 and 30. Antibodies are usually detected in the eggs by day 30.

Eggs are collected, the yolk separated from the white, and the immunoglobulin fraction is isolated from the egg yolk. As a first step, the lipids and lipoproteins are removed, using a suitable method known to persons skilled in the art, for example precipitation using PEG or dextran sulphate, solubilisation using organic solvents, or ultrafiltration. The resulting substantially lipid-free solution can then be concentrated and purified to obtain the desired immunoglobulin fraction containing polyclonal antibodies against the antigen. Methods for the concentration and/or purification of immunoglobulins are also well known to a person skilled in the art, and include precipitation steps using for example PEG or sodium sulphate, or other well known methods such as ultrafiltration or liquid chromatography.

### Calibrators

Calibrators are produced by diluting purified calprotectin in a suitable buffer at pH 7.4 to achieve different calibration levels. Examples of buffers include phosphate buffered saline (PBS) and HEPES buffer, both available from commercial sources (e.g. Sigma-Aldrich Norway AS, Oslo, Norway). The calprotectin concentration of the stock solution is then determined, for example by the Biuret method (BioquantTM, Merck KGaA, Darmstadt, Germany).

### Controls

Controls are prepared by adding purified calprotectin to human serum. Two levels are usually prepared, for example 1.0 and 10 mg/L depending on the diagnostic range targeted in the assay.

### Particle enhanced immunoassay

The assay can be operated manually, but is preferably run on an automated clinical chemistry instrument, such as the MindrayTM BS-380 (Mindray Medical International, Shenzhen, China) or the Abbott Architect c4000 (Abbott Diagnostics, USA) to mention two non-limiting examples. A person skilled in the art can optimize the parameter settings for the assay on any suitable automated clinical chemistry instrument. Representative parameter settings are shown in Table 2:

**Table 2. An example of PETIA parameters on an automated instrument**

| **Parameter** | **Setting** |
|---|---|
| Sample volume [µL] | 3 |
| R1: Assay buffer volume [µL] | 200 |
| R2: Reagent volume [µL] | 30 |
| Wavelength [nm] | 605 |
| Reading time 38/39-55/56 [s] | 204 |
| Cycle length [s] | 12 |

The improved assay methods and kits disclosed herein have many advantages. They are more flexible, and yet more reliable and repeatable, and substantially independent of the choice of sample treatment method and reagents. A turbidimetric assay is well suited fro automation and can be run on many commercially available autoanalyzers. By adjusting the settings, and possibly including alternative reagents, the assay can be adapted to different sample material.

The assay method and kit is/are applicable to plasma, serum and faeces samples alike, and it is conceived that the robustness of the method makes it applicable also to other samples, such as for example cerebrospinal fluid and synovial fluid. The assay is possibly also applicable to biopsy samples and faecal samples, which following homogenization and extraction can be run on automated clinical instruments, such as those exemplified above,

### Examples

### Example 1. Comparison of patient samples

The objective of this study was to investigate if the measured calprotectin concentration in serum and plasma from the same patient is dependent on the sample material used (EDTA plasma, lithium heparin plasma or serum).

### 1.1 Materials and methods

22 patients were included, and blood samples drawn in commercial sample collection tubes according to standard procedures as follows:
- Serum, lithium heparin and EDTA samples: 9 patients;
- EDTA plasma and lithium heparin plasma: 12 patients; and
- lithium heparin plasma and serum: 1 patient.

The samples were collected at the Karolinska Hospital, Stockholm, Sweden and frozen before shipment to Gentian AS, Moss, Norway. The samples were thawed in lukewarm water bath for 10 minutes, centrifuged for 2 minutes and assayed in duplicates at the Gentian facilities.

All samples were assayed using a particle enhanced turbidimetric immunoassay (Gentian Calprotectin PETIA, Gentian AS, Norway) in duplicates on an automated clinical chemistry instrument (Abbott Architect c4000, Abbott Diagnostics, USA) according to manufacturer's instructions but using optimised parameter. See Table 3 below.

In the current assay, the immunoparticles were prepared by covalently attaching purified avian immunoglobulin fractions to uniform polystyrene particles. The avian immunoglobulin fractions were purified from eggs from immunized hens. The antibodies were directed against native human calprotectin (a heterotetramer of MRP8/MRP14 complexes). Calprotectin extracted and purified from human granulocytes was used in the process of raising the avian antibodies. The reaction buffer was a 3-(N-morpholino) propanesulfonic (MOPS) buffer with pH in the interval 6.9 - 7.2.

Calibrators were produced by diluting purified calprotectin in a HEPES buffer at pH=7.4 to achieve six calibration levels in the interval 0 -19 mg/L.

Controls were prepared by diluting one of the calibrators at two levels; 1 mg/L and 10 mg/L.

The parameter settings for the automated clinical chemistry instrument (Abbott Architect c4000) were optimised for the Gentian Serum Calprotectin Immunoassay. The main parameter settings are shown in Table 3.

**Table 3. Optimised PETIA parameter settings**

| **Parameter** | **Setting** |
|---|---|
| Sample volume [µL] | 4 |
| R1: Assay buffer volume [µL] | 180 |
| R2: reagent volume [µL] | 30 |
| Primary wavelength [nm] | 604 |
| First reading time [cycle] | 12-15 |
| Second reading time [cycle] | 21-23 |

### 1.2 Results

The results show that there is a difference between the calprotectin concentrations determined in the different samples although they originate from the same patient. The most striking result is that the EDTA treated samples exhibited a much lower concentration when analysed in the PETIA. There was also a difference between lithium heparin and serum sample, most apparent at lower concentrations. This difference was even more pronounced when the samples are fresh, compared to the same samples when analysed after having been frozen.

Table 4 shows the measured calprotectin concentration (mg/L) of the 22 patients and corresponding sample materials.

Table 5 shows a statistical summary for each of the sample materials, excluding sample 21, demonstrating a difference between the sample materials based on measured calprotectin concentration.

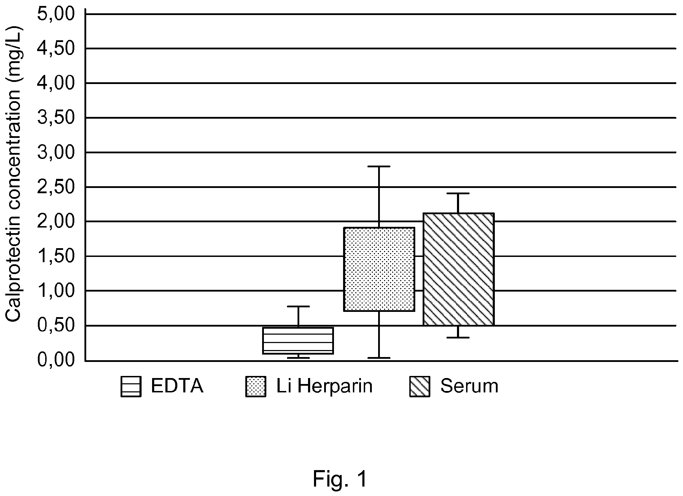

Figure 1 shows a boxplot for each of the sample materials, excluding sample 21, demonstrating a difference between the sample materials EDTA, lithium heparin and serum, based on measured calprotectin concentration.

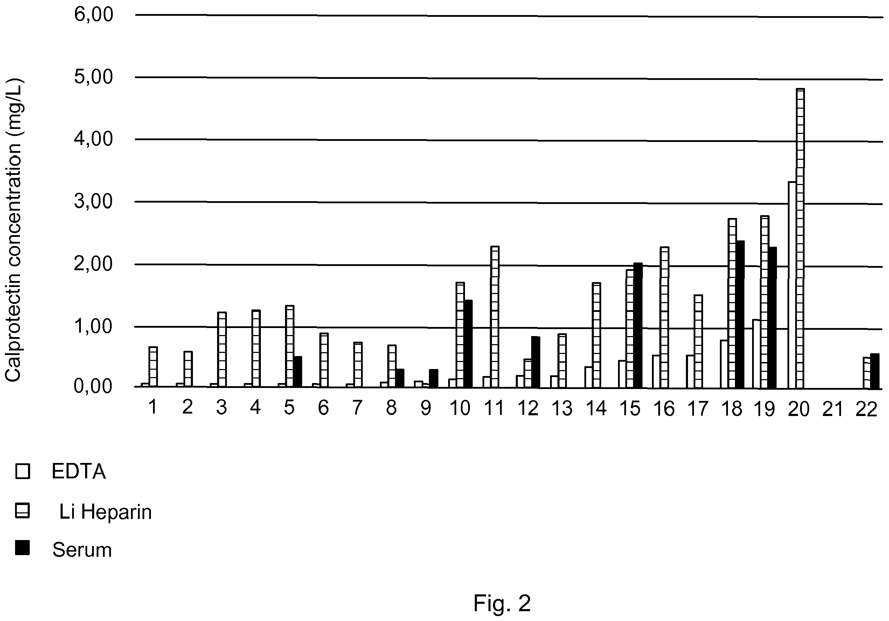

Figure 2 shows a bar plot of each individual patient and sample material, excluding sample 21.

**Table 4: Measured calprotectin concentration (mg/L) in EDTA plasma, Li Heparin plasma and Serum in 22 different patients**

| | Mean calprotectin concentration (mg/L) | | |
|---|---|---|---|
| Sample ID | **EDTA** | **Li Heparin** | **Serum** |
| 1 | 0,04 | 0,67 | |
| 2 | 0,04 | 0,58 | |
| 3 | 0,04 | 1,23 | |
| 4 | 0,05 | 1,24 | |
| 5 | 0,06 | 1,32 | 0,51 |
| 6 | 0,06 | 0,87 | |
| 7 | 0,07 | 0,74 | |
| 8 | 0,09 | 0,69 | 0,31 |
| 9 | 0,13 | 0,04 | 0,32 |
| 10 | 0,15 | 1,72 | 1,42 |
| 11 | 0,19 | 2,29 | |
| 12 | 0,21 | 0,45 | 0,84 |
| 13 | 0,22 | 0,88 | |
| 14 | 0,36 | 1,69 | |
| 15 | 0,44 | 1,93 | 2,03 |
| 16 | 0,53 | 2,31 | |
| 17 | 0,55 | 1,51 | |
| 18 | 0,77 | 2,74 | 2,39 |
| 19 | 1,12 | 2,79 | 2,32 |
| 20 | 3,34 | 4,82 | |
| 21* | 68 | 70 | |
| 22 | | 0,53 | 0,57 |

| | | | |
|---|---|---|---|
| *Sample excluded from analysis. | | | |

**Table 5: Summary statistics of calprotectin concentration (mg/L) in EDTA plasma, Li Heparin plasma and Serum in 21 different patients**

| | **EDTA** | **LithiumHeparin** | **Serum** |
|---|---|---|---|
| Mean | 0,42 | 1,48 | 1,19 |
| Standard Error | 0,17 | 0,24 | 0,29 |
| **Median** | **0,17** | **1,24** | **0,84** |
| Standard Deviation | 0,75 | 1,09 | 0,86 |
| Range | 3,31 | 4,78 | 2,08 |
| Minimum | 0,04 | 0,04 | 0,31 |
| Maximum | 3,34 | 4,82 | 2,39 |
| Sum | 8,42 | 30,98 | 10,69 |
| Count | 20,00 | 21,00 | 9,00 |

The results show that the sample collection method influences the results, and demonstrates the need for a more robust assay, or a need for assay steps that take the type of sample into account or compensates for the differences.

### Example 2. Investigation of calprotectin isoforms in different samples

The objective was to investigate if calprotectin exhibited different isoforms in samples subjected to different treatment (serum, lithium-heparin plasma, and EDTA) and also to compare to the isoform present in the calibrator. To this end, Size Exclusion Chromatography (SEC) experiments were performed and combined with turbidimetric measurements of the fractions collected after separation on SEC column.

### 2.1 Materials and methods

### 2.1.1 Chemicals

Unless otherwise stated all reagents and chemical compounds as used herein were of analytical grade. All chemicals were supplied by Sigma-Aldrich. The turbidimetric reagents were developed by Gentian AS.

### 2.1.2 Instruments

The calprotectin concentration was assessed by using a modified Gentian particle enhanced turbidimetric immunoassay on a fully automated clinical chemistry instrument Mindray™ BS-380 (Mindray Medical International, Shenzhen, China). The parameter settings were as shown in Table 6.

**Table 6. Modified PETIA settings**

| **Parameter** | **Setting** |
|---|---|
| Latex particles size (nm) | 90 - 150 |
| R1: Assay buffer Volume [µL] | 150 |
| Sample Volume [µL] | 2 - 15 |
| R2: Immunoparticles [µL] | 30 |
| First reading time [cycle] | 37-37 |
| Second reading time [cycle] | 52-52 |
| Primary wavelength [nm] | 605 |

The protein concentration of the samples was determined by optical density (OD) measurements at 280nm using a Thermo Scientific NanoDrop 2000 spectrophotometer. The molar extinction coefficient for calprotectin used to determine the concentration was 0.76 L/g cm.

The Size Exclusion Chromatography experiments were performed at two different locations with different columns. In total, three instruments were used:
- Äkta™ Explorer 100 system (GE Healthcare Lifesciences, Sweden) at the first location
- Äkta™ Purifier 900 system (GE Healthcare Lifesciences, Sweden) at the second location
- Agilent 1260 Infinity Quaternary LC system (Agilent Technologies, USA) at the second location

Three different columns were used:
- A Superdex™ 75 10/300 GL column (GE Healthcare) was used to pre-purify the patient samples,
- An AdvanceBio SEC 300 Å 2.7µm 4.6x300mm column (Agilent Technologies) was used to isolate the protein of interest before molecular weight investigation
- An AdvanceBio SEC 300 Å 2.7µm 4.6x150mm column (Agilent Technologies) was used to investigate and determine the molecular weight of the isolated proteins from patient samples and the molecular weight of the purified antigens.

### 2.1.3 Calprotectin samples

Calprotectin from three different sources was investigated:
a. Recombinant calprotectin was purchased from ImmunDiagnostik AG, Germany, and from Yashraj Biotechnology Ltd, India. The samples were delivered in solution.
b. Native calprotectin extracted from buffy coats isolated from donor blood from healthy donors. The buffy coats were isolated by staff at Ullevål University Hospital, Oslo, and the extraction performed at Gentian AS as described below.
c. Patient samples collected as EDTA plasma, lithium heparin plasma and serum samples were obtained from Rikshospitalet, Oslo, Norway (EDTA plasma samples) and from Uppsala University Hospital, Uppsala, Sweden (lithium heparin plasma and serum samples).

### 2.2 Methods

In this example, two different categories of samples were analyzed: purified proteins (recombinant and native calprotectin) and physiological samples, drawn from patients. Typically, purified proteins are available in 95-99% pure form, containing only the protein of interest in its different isoforms.

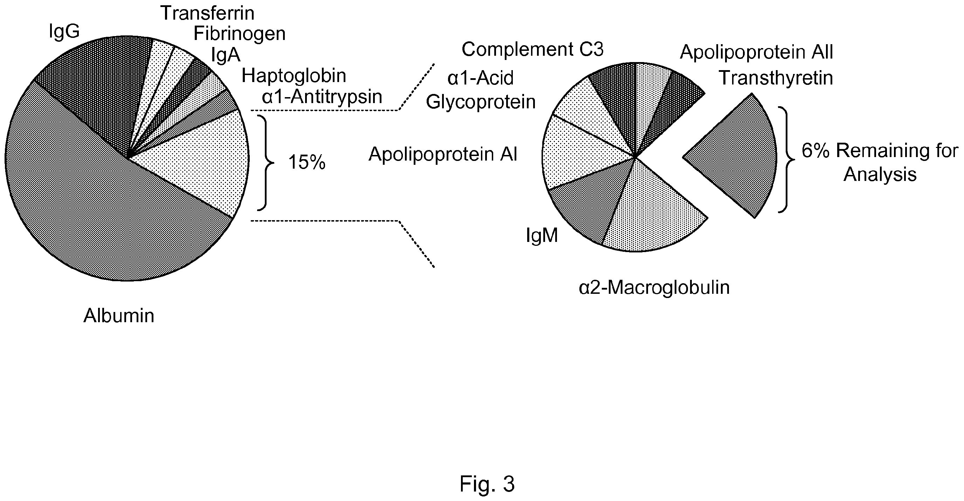

In patient samples the protein of interest is however only available in a low concentration, together with all the circulating proteins present in the blood, particularly the most abundant proteins (serum albumin, IgG, transferrin, fibrinogen, IgA, haptoglobin, etc), as illustrated in Figure 3 (Source: Mrozinski et al., Human Serum and Plasma Protein Depletion - Novel High-Capacity Affinity Column for the Removal of the "Top 14" Abundant Proteins; Agilent Technologies).

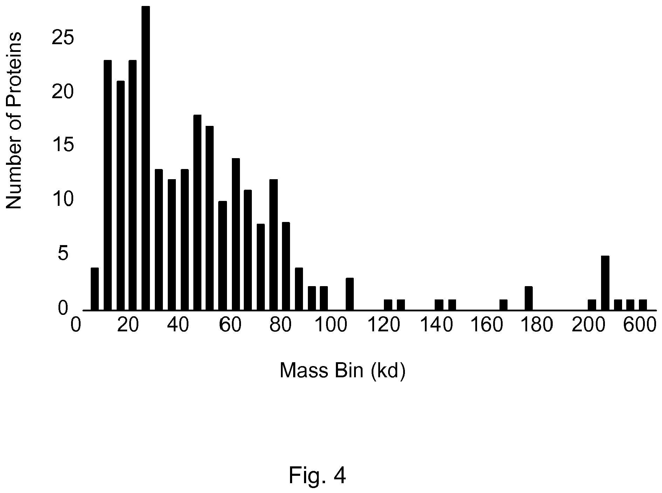

In the plasma and serum proteomes, a majority of proteins are found in the molecular weight range up to 90 -100 kDa. For an illustration, see Figure 4 showing a histogram of the calculated masses of the processed forms of 289 proteins observed in plasma (Source: Anderson and Anderson, The Human Plasma Proteome, History, Character and Diagnostic Prospects, Molecular & Cellular Proteomics, 1.11, 2002, page 845-867).

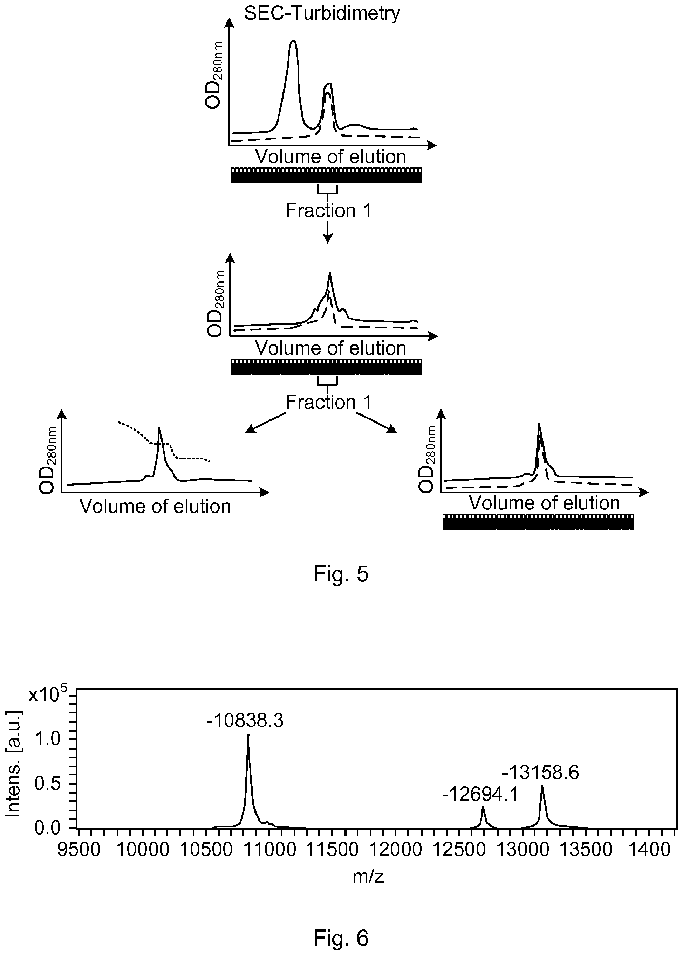

Based on the calculated models for the different isoforms of calprotectin, calprotectin can be present in its main forms from 10.8 to 52.8 kDa (Table 1). It is known that calprotectin can also form complexes of higher molecular weight isoforms by aggregation, most generally due to the presence of bivalent cations. This demonstrates that analyzing calprotectin in patient samples is a challenge, since the calprotectin is difficult to isolate from the samples. Therefore, series of purification on different materials, such as EDTA plasma, Li-Heparin plasma, and serum were performed as illustrated in Figure 5.

### 2.3 Results

In serum and lithium heparin plasma samples, similar profiles were observed in the SEC experiments, and the molecular weight of approximately 68 to 81 kDa indicates the presence of hexamers or heptamers of calprotectin. The results are summarized in Table 7.

**Table 7. Molecular weights indicated in SEC-MALLS/RI analysis**

| **Samples** | **Major peak** | **Minor peak** | **Other peaks** |
|---|---|---|---|
| S96 | 72kDa | 160kDa | 237kDa |
| LiH78 | 69.5kDa | 160kDa | nd |
| | 81.6kDa | 170kDa | nd |
| LiH17-2a | 74kDa | 160kDa | >700kDa |
| LiH17-2b | 73kDa | 172kDa | >450kDa |
| | | | >1MDa |
| LiH17-2c | 68kDa | 175kDa | >450kDa |
| LiH17-2d | 81 kDa | 360kDa | >1MDa |

Regarding the antigen, it was shown that tetramers were present in the native antigen, extracted from donor blood. The recombinant form, supposed to be tetrameric, was shown to be pentameric and hexameric. Interstingly, the matrix of those samples contains bivalent cations, which constitutes a considerable difference compared to the native antigen, in saline. The results are summarized in Table 8.

**Table 8. SEC-MALLS/RI analysis of calprotectin from different sources**

| **Conditions** | **Native calprotectin** | **Recombinant calprotectin** | |
|---|---|---|---|
| | Gentian AS | ImmunDiagnostik AG | Yashraj Biotechnology Ltd |
| Control | 45,5 - 49,5kDa | Lot1: 59kDa | 62kDa |
| | 120kDa or 160 - 170kDa | Lot2: 69kDa | 155kDa / 610kDa |
| + EDTA | 1 h incubation at RT: 76,71,48,and 31kDa | 1h incubation at RT: 130kDa (Lot1) | nd |
| | 20h incubation at RT: 124, 33,6kDa 40h incubation | | |
| | at RT: >500, 39kDa | | |
| + CaCl₂ | 1h incubation at RT: 500-610, 56kDa | 1h incubation at RT: 100kDa (Lot2) | nd |
| | 40h incubation at RT: >550,102kDa | | |

Several lots of native calprotectin were tested over time in order to assess the stability of calprotectin. The results (not shown) indicated that the native calprotectin was indeed very stable. As shown in Figure 6, the mass spectrometry results indicate the presence of the truncated form of S100A9.

The recombinant antigens, produced in *E. Coli,* have a molecular weight corresponding to a complex heavier than a tetramer: pentameric (59kDa) and hexameric (69kDa) isoforms. This is believed to be due to the manner in which calprotectin is produced. Both recombinant single chain of calprotectin are produced in parallel and individual bacterial cultures. When the recombinant products are obtained and purified, they are mixed together to form the calprotectin complex. Because the bacterial environment is different from the human one, the recombinant proteins will not be completed in terms of post-translational modifications (phosphorylation, glycosylation, etc.) and maturation. Subsequently, the complex formation is not controlled and can lead to the production of various types of complex, different from the ones found naturally. It is also possible that the production of recombinant calprotectin is performed with a combined DNA vector, a chimera, coding for both proteins in a same and unique gene. The resulting product will be able to associate in higher molecular weight isoforms than a hetero-dimer, but never lower. This could explain the fact that neither calcium nor EDTA had the expected effect on these recombinant isoforms.

The native calprotectin isolated and purified from granulocytes is subjected to EDTA and calcium during the purification process. However, a molecular weight for each lot was estimated to between 45.5 and 49.5kDa. This clearly shows that the native form is tetrameric (the expected molecular weight is 48kDa).

The tetrameric form of calprotectin was found to be sensitive to calcium. An addition of calcium results in aggregation of the calprotectin, forming higher molecular weight species up to 100kDa.

The tetrameric form of calprotectin is also sensitive to EDTA, a chelating agent for bivalent cations. The addition of EDTA however results in a slow process of dissociation, resulting in a minimum observed trimeric isoform of approximately 35kDa. A dissociation between a metal and its ligand has usually a enthalpy much greater than its corresponding association, most particularly when a metal is binding residues in the well-described HEXXHXXXXXH motif in proteins. Thus, the process of chelation of Ca²⁺ by EDTA is slow. In the experiments performed so far, an effect could be seen after one hour incubation at room temperature. A stabilized effect was seen after 40 hours of incubation. Interestingly, the native antigen is also tetrameric, with a presence of an entity around two- to three-fold its main molecular weight, which could be interpreted as an agglomerate in an octa- or dodecamer isoform.

The patient samples required repeated purification steps. After two size exclusion steps and isolation of "fraction 2" as described in Fig. 5, a common major peak between 70 and 80kDa was detected. This indicates the presence of a hexamer of calprotectin. A minor peak was observed as well between 160 and 175kDa, which could illustrate the presence of a dodecamer of calprotectin, as observed in native calprotectin (Table 8).

All these results correspond to UV peaks detected in the SEC-MALLS/RI analysis, and have a clear match with the corresponding calprotectin turbidimetric signals. This indicates the presence of calprotectin in the peaks corresponding to the two above-mentioned molecular weights.

### Further experiments

The behavior of these isoforms will be monitored by the addition of EDTA/EGTA or CaCl₂/MgCl₂ to the samples. The reaction kinetics will be investigated. The current results and observations indicate that an addition of EDTA results in a clear shift, but that the dissociation of calprotectin complexes is very slow. Conversely, an addition of an excess of bivalent cations results in a rapid formation of higher isoforms.

In order to confirm the identity of the proteins, samples will be sent for analysis by Mass Spectrometry to confirm the identity of the proteins for which SEC-MALLS/RI determined molecular weight indicating different isoforms of calprotectin.

Without further elaboration, it is believed that a person skilled in the art can, using the present description, including the examples, utilize the present invention to its fullest extent. Also, although the invention has been described herein with regard to its preferred embodiments, which constitute the best mode presently known to the inventors, it should be understood that various changes and modifications as would be obvious to one having the ordinary skill in this art may be made without departing from the scope of the invention which is set forth in the claims appended hereto.

Thus, while various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following claims.

## Claims

1. A method for determination of calprotectin in a sample using a particle enhanced turbidimetric immunoassay (PETIA), said assay comprising anti-calprotectin antibodies or fragments thereof, immobilized to particles, a reaction buffer, and a calibrator comprising calprotectin in a buffer; **characterized in that** the choice of calibrator buffer and/or reaction buffer is determined by the character of the sample.

2. The method according to claim 1, wherein the sample is a lithium heparin treated plasma sample or a serum sample and the calibrator buffer and/or reaction buffer comprises an excess of bivalent cations.

3. The method according to claim 1, wherein the sample is an EDTA treated sample and the calibrator buffer and/or reaction buffer comprises EDTA.

4. The method according to claim 3, wherein the sample is a fecal extract and the calibrator buffer and/or reaction buffer comprises EDTA.

5. A method for determination of calprotectin in a sample using a particle enhanced turbidimetric immunoassay (PETIA), said assay comprising anti-calprotectin antibodies or fragments thereof, immobilized to particles, a reaction buffer, and a calibrator comprising calprotectin in a buffer; **characterized in that** said calibrator buffer comprises EDTA and/or that EDTA is added to the sample before or simultaneously with the addition of the reaction buffer.

6. A method for determination of calprotectin in a sample using a particle enhanced turbidimetric immunoassay (PETIA), said assay comprising anti-calprotectin antibodies or fragments thereof, immobilized to particles, a reaction buffer, and a calibrator comprising calprotectin in a buffer; **characterized in that** said calibrator buffer comprises bivalent cations and that an excess of bivalent cations is added to the sample before or simultaneously with the addition of the reaction buffer.

7. The method according to one of claims 1, 5 and 6, wherein said anti-calprotectin antibodies or fragments thereof are antibodies against purified S100A8/A9 heterotetramers extracted from human granulocytes.

8. The method according to one of claims 1, 5 and 6, wherein said anti-calprotectin antibodies or fragments thereof are antibodies against recombinant calprotectin.

9. The method according to one of claims 1, 5 and 6, wherein said particles are polystyrene particles having a particle size in the interval of 60 - 180 nm, preferably 60 - 120 nm, most preferably 80 - 100 nm in diameter.

10. The method according to one of claims 1, 5 and 6 wherein said reaction buffer is chosen from phosphate buffered saline (PBS), 3-(N-morpholino) propanesulfonic (MOPS) buffer and (4-(2-hydroxyethyl)-1-piperazine ethanesulfonic acid (HEPES) buffer.

11. A kit for use in the method of claim 1, comprising one or more anti-calprotectin antibodies or fragments thereof immobilized on particles, a reaction buffer, and a calibrator comprising calprotectin in a buffer, **characterized in that** the reaction buffer is chosen from phosphate buffered saline (PBS), 3-(N-morpholino) propanesulfonic (MOPS) buffer and (4-(2-hydroxyethyl)-1-piperazine ethanesulfonic acid (HEPES) buffer, and **in that** the calibrator buffer and/or reaction buffer comprises EDTA when the sample is an EDTA treated sample.

12. The kit according to claim 11, wherein the calibrator buffer comprises EDTA when the sample is a fecal extract.

13. A kit for use in the method of claim 5, comprising one or more anti-calprotectin antibodies or fragments thereof immobilized on particles, a reaction buffer, and a calibrator comprising calprotectin in a buffer, **characterized in that** the reaction buffer is chosen from phosphate buffered saline (PBS), 3-(N-morpholino) propanesulfonic (MOPS) buffer and (4-(2-hydroxyethyl)-1-piperazine ethanesulfonic acid (HEPES) buffer, and **in that** the reaction buffer and/or the calibrator buffer comprise EDTA.

14. A kit for use in the method of claim 6, comprising one or more anti-calprotectin antibodies or fragments thereof immobilized on particles, a reaction buffer, and a calibrator comprising calprotectin in a buffer, **characterized in that** the reaction buffer is chosen from phosphate buffered saline (PBS), 3-(N-morpholino) propanesulfonic (MOPS) buffer and (4-(2-hydroxyethyl)-1-piperazine ethanesulfonic acid (HEPES) buffer, and **in that** the reaction buffer and/or the calibrator buffer comprise an excess of bivalent cations.

15. The kit according to any one of claims 11 to 14, wherein said anti-calprotectin antibodies or fragments thereof are antibodies against purified S100A8/A9 heterotetramers extracted from human granulocytes.

16. The kit according to any one of claims 11 to 14, wherein said anti-calprotectin antibodies or fragments thereof are antibodies against recombinant calprotectin.

17. The kit according to any one of claims 11 to 14, wherein said particles are polystyrene particles having a particle size in the interval of 60 - 180 nm, preferably 60 - 120 nm, most preferably 80 - 100 nm in diameter.
